# EUROPEAN PATENT SPECIFICATION

(11) **EP 3 183 387 B1**
(45) Date of publication and mention of the grant of the patent: **25.09.2019**
(21) Application number: 14900111.7
(22) Date of filing: 19.08.2014
(51) Int. Cl.: D06M 14/18, A61F 2/08, A61F 2/10, A61L 27/00, D06M 15/285, D06M 15/356

(54) **COMPOSITE COMPRISING FABRIC AND POLYAMPHOLYTE HYDROGEL AND PREPARATION METHOD THEREOF**
VERBUNDSTOFF MIT GEWEBE UND POLYAMPHOLYT-HYDROGEL UND HERSTELLUNGSVERFAHREN DAFÜR
COMPOSITE COMPRENANT UN TISSU ET UN HYDROGEL DE POLYAMPHOLYTE, ET PROCÉDÉ DE PRÉPARATION ASSOCIÉ

(43) Date of publication of application: 28.06.2017
(73) Proprietor: National University Corporation Hokkaido University, Sapporo-shi, Hokkaido 060-0808 (JP); University of Massachusetts, Boston, MA 02110 (US)
(72) Inventor: GONG, Jian Ping, Sapporo-shi Hokkaido 060-0808 (JP); KUROKAWA, Takayuki, Sapporo-shi Hokkaido 060-0808 (JP); SUN, Tao Lin, Sapporo-shi Hokkaido 060-0808 (JP); KING, Daniel, Boston, MA 02108 (US); CROSBY, Alfred, Boston, MA 02108 (US)
(74) Representative: Palladino, Saverio Massimo
(86) International application number: PCT/JP2014/072080
(87) International publication number: WO 2016/027383

(56) References cited:
- JP-A- 2005 169 112
- JP-A- 2005 526 879
- JP-A- 2006 503 118
- JP-A- 2011 072 241
- JP-A- 2013 514 422
- US-A1- 2006 183 822
- ZHIFENG ZHU ET AL: "Effect of amphoteric grafting branch on the adhesion of starch to textile fibers", JOURNAL OF ADHESION SCIENCE AND TECHNOLOGY, vol. 28, no. 17, 30 April 2014 (2014-04-30), pages 1695-1710, XP055445425, GB ISSN: 0169-4243, DOI: 10.1080/01694243.2014.913514
- XIAOGUANG MA ET AL: "Modifying terylene/cotton blend fabric with P(AMPS/NIPA) intelligent hydrogel by graft polymerization initiated with MLTP", THE JOURNAL OF THE TEXTILE INSTITUTE, TAYLOR & FRANCIS, GB, vol. 105, no. 8, 24 January 2014 (2014-01-24), pages 878-886, XP001589076, ISSN: 0040-5000, DOI: 10.1080/00405000.2013.860694 [retrieved on 2014-01-24]
- TAO LIN SUN ET AL: "Physical hydrogels composed of polyampholytes demonstrate high toughness and viscoelasticity", NATURE MATERIALS, vol. 12, no. 10, 28 July 2013 (2013-07-28) , pages 932-937, XP055445406, GB ISSN: 1476-1122, DOI: 10.1038/nmat3713
- AGRAWAL A ET AL: "Strong fiber reinforced hydrogels for biomedical applications", BIOENGINEERING CONFERENCE (NEBEC), 2011 IEEE 37TH ANNUAL NORTHEAST, IEEE, 3 April 2011 (2011-04-03), pages 1-2, XP031873789, DOI: 10.1109/NEBC.2011.5778724 ISBN: 978-1-61284-827-3
- MOUTOS F T ET AL: "A biomimetic three-dimensional woven composite scaffold for functional tissue engineering of cartilage", NATURE MATERIALS, NATURE PUBLISHING GROUP, GB, vol. 6, no. 2, 21 January 2007 (2007-01-21), pages 162-167, XP002596216, ISSN: 1476-1122, DOI: 10.1038/NMAT1822 [retrieved on 2007-01-21]
- LUYI WANG ET AL: "A strong and tough interpenetrating network hydrogel with ultrahigh compression resistance", SOFT MATTER, vol. 10, no. 21, 14 April 2014 (2014-04-14), page 3850, XP055445412, ISSN: 1744-683X, DOI: 10.1039/c4sm00206g

## Description

### Background of The Invention

### Field of The Invention

This invention relates to a composite comprising a fabric and a polyampholyte hydrogel and a method of preparation of the composite.

### Discussion of The Background

Anterior Cruciate Ligament (ACL) reconstruction surgeries are one of the most common surgeries performed by orthopedic surgeons. To avoid using autographs or allographs, synthetic materials have been investigated as potential replacements.

Some materials which have been tested are Dacron, Marlex, Teflon, Nylon, and Carbon Fiber.[NP-2] It is important that the materials which are used can support the high load placed upon these ligaments. Carbon Fiber has been especially of interest as a replacement material because of its extremely high stiffness. Initial results with neat carbon fiber replacements were promising.[NP-3,4] However problems were encountered, including fracture, fraying and spreading of carbon in the body, irritation and poor healing of the surrounding area, and accumulation of carbon in regional lymph nodes.[NP-1,2,5]
NP-1: K. Miller, J. E. Hsu, L. J. Soslowsky, in Comprehensive Biomaterials, 2011, pp.257-279.
NP-2: A. A. Amis, S. A. Kempson, J. R. Campbell, J. H. Miller, Journal of Bone and Joint Surgery 1988, 70-B, 628-634.
NP-3: D. H. R. Jenkins, B. McKibbin, Journal of Bone and Joint Surgery 1980, 62, 497-499.
NP-4: D. H. R. Jenkins, Journal of Bone and Joint Surgery 1978, 60-B, 520-522.
NP-5: S. Ramakrishna, J. Mayer, Composites Science and ... 2001, 61, 1189-1224.

Some researchers have also attempted to use hydrogels as ligament replacements, but they lack the mechanical properties to support high loads. There is some mention in the patent literature of hydrogel and fabrics as prosthetics. [P-1, 2, 3, 4]
P-1: US Patent No. 6,629,997.
P-2: US Patent No. 6,187,043.
P-3: US Patent No. 6,027,744.
P-4: US Patent No. 4,919,667.

However the brittleness of traditional hydrogels may have prevented these methods from entering the market.

In addition to the application in ACL reconstruction surgeries, the applications listed below are those in which high toughness is desired, such as skin grafts, flexible tear resistant clothing, bandaging, bullet-proofing, etc.

In bandages, there is very little in the literature about fabric / hydrogel bandages, however there is some mention in the patent literature. In these systems fabric are used as support, and the hydrogel is used for wound closure, as they are generally non-toxic, consist mostly of water, and can promote wound healing. [P-5 to P-8]
P-5: Absorptive wound dressing for wound healing promotion - 5,695,777 ;
P-6: Dressing material based on a *hydrogel,* and a process for its production - 4,552,138 ;
P-7: Reinforced, laminated, impregnated, and composite-like materials as crosslinked polyvinyl alcohol *hydrogel* structures - 6,855,743;
P-8: Hydrogel Laminate, Bandages, and composites and methods for forming the same - 5,674,346

However, within these patents there is no obvious mention of utilizing these composite materials to make extremely tough or tear resistant bandages.

Fabric composites have been looked at and utilized before as high strength and toughness materials. Some of these are for ballistics and blast resistance.
P-9: Reinforced film for blast resistance protection - 8,039,102;
P-10: Impact resistive composite materials and methods for making same - 7,608,322;
P-11: Body armor with improved knife-stab resistance formed from flexible composites - 7,288,493;
P-12: Ballistic-resistant article and process for making the same - 6,268,301;
P-13: Ballistic *fabric* laminates - 6,846,758;
P-14: Armor panel - 5,789,327;
P-15: Method for improving the energy absorption of a high tenacity *fabric* during a ballistic event - 5,595,809;
P-16: Lightweight armor and ballistic projectile defense apparatus - 8,375,839).

There are materials used for airbag technologies in vehicles.
P-17: Silicone composition for coating substrates made of textile material - 6,586,551;
P-18: Silicone coated textile fabrics - 6,354,620; etc.

Finally, some patents mention fabrics which exhibit high tear strength.
P-19: Textile-reinforced composites with high tear strength - 7,549,303;
P-20: Method of making a cut and abrasion resistant laminate - 6,280,546;
P-21: Elastic composite structure - 7,153,789;
P-22: Composite elastic material - 6,706,364;
P-23: Cut resistant yarn, *fabric* and gloves - 5,119,512;
P-24: Fiber reinforced thermosetting resin compositions with coated fibers for improved toughness - 4,737,527;
P-25: Method of manufacture of silicone elastomer coated cloth - 4,478,895.

However in these examples, none of the composite materials utilize hydrogels, or any type of elastic materials which contain water. In many cases, the matrix material is thermosetting, which would result in the composite lacking any flexibility.

Recently, new polyampholyte (PA) hydrogels produced by Sun et. al. have shown the ability to maintain these features through a macromolecular architecture that also features self-healing, and one-pot synthesis abilities.[NP-6,7]
NP-6: T. L. Sun, T. Kurokawa, S. Kuroda, A. Bin Ihsan, T. Akasaki, K. Sato, M. A. Haque, T. Nakajima, J. P. Gong, Nature materials 2013, 12, 1 -6, and T. L. Sun, T. Kurokawa, S. Kuroda, S, A. B. Ihsan, T. Akasaki, K. Sato, Md. A. Haque, T. Nakajima and J. P. Gong, Nature materials, 2013, 12, 932-937
NP-7: A. Bin Ihsan, T. L. Sun, S. Kuroda, M. A. Haque, T. Kurokawa, T. Nakajimac, J. P. Gong, J. Mater. Chem. B, 2013, 1, 4555-4562

Previous work from the Crosby Research Group has shown that embedding soft elastomers with stiff fabrics can result in very interesting composite materials.[NP-8 to NP-10] Double network hydrogels devolved in the Gong Research Group have extremely high toughness, while having moderate ultimate tensile strength and low friction. [NP11-14].

NP-8: M. D. Bartlett, A. B. Croll, D. R. King, B. M. Paret, D. J. Irshick, A. J. Crosby, Advanced Materials 2012, 24, 1078-1083.
NP-9: S. A. Pendergraph, M. D. Bartlett, K. R. Carter, A. J. Crosby, ACS Applied Materials & Interfaces 2012, 4, 6640-5.
NP-10: D. R. King, M. D. Bartlett, C. A. Gilman, D. J. Irschick, A. J. Crosby, in prep 2013.
NP-11: J. P. Gong, Y. Katsuyama, T. Kurokawa, Y. Osada, Advanced Materials 2003, 15, 1155-1158. Revised 12/17/12 4
NP-12: J. P. Gong, T. Kurokawa, T. Narita, G. Kagata, Y Osada, G. Nishimura, M. Kinjo, Journal of the American Chemical Society 2001, 123, 5582-5583.
NP-13: T. Tominaga, N. Takedomi, H. Biederman, H. Furukawa, Y. Osada, J. P. Gong, Soft Matter 2008, 4, 1033.
NP-14: K. Yasuda, J. P. Gong, Y. Katsuyama, A. Nakayama, Y Tanabe, E. Kondo, M. Ueno, Y Osada, Biomaterials 2005, 26, 4468-4475.

US 2006/068014 A1 (P-25) discloses a composite obtained by coating a pre-polymerisation mix onto a substrate, preferably siliconised, wherein the substrate can be a non-woven material made of natural and/or synthetic materials and by curing under UV light, so that an hydrogel film having a thickness from 0.05 mm to 3 mm is formed. The hydrogel exhibits polyampholyte rather than polyelectrolyte behaviour. The pre-polymerisation mix contains a mixture a first monomer comprising one or more pendant anionic group and a second monomer comprising one or more pendant cationic group, wherein the first monomer is 3-sulphopropyl acrylate (SPA) or a salt or analogue thereof, 2-acrylamido-2-methylpropane sulphonic acid (AMPS) or a salt or analogue thereof, or a mixture of both and the second monomer is acryloyloxyethyltrimethyl ammonium chloride, acryloyl-oxyethyltrimethyl ammonium methyl sulphate, or acrylamidopropyltrimethyl ammonium chloride.

US 2006/183822 A1 (P-26) discloses an ampholytic copolymer obtainable by free-radical copolymerization of a) at least one compound with a free-radically polymerizable, α, β-ethylenically unsaturated double bond and at least one anionogenic and/or anionic group per molecule.

Utilizing a fabric and PA hydrogel composite could allow for high loads (from the fiber) while the PA hydrogel could prevent wear and rejection from the body. Incorporating these new hydrogels into fabric will allow for new materials with unique properties that are currently lacking in the literatures and patents.

### Summary of The Invention

According to a first aspect the present invention discloses a composite comprising a fabric and a polyampholyte hydrogel, wherein the polyampholyte hydrogel is a hydrogel of a polymer bearing randomly dispersed cationic and anionic repeat groups; and at least a part of the fabric is coated with the polyampholyte hydrogel, wherein the polyampholyte hydrogel is selected from the group consisting of random polymers obtained by random polymerization of at least one cationic monomer selected from the group consisting of 3-(methacryloylamino)propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC) and the anionic monomer sodium 4-styrenesulfonate (NaSS), and wherein the molar ratio of the anionic monomer to the total of the anionic monomer and a cationic monomer is in a range from 0.4 to 0.6, and the total concentration of the anionic and the cationic monomer in a monomer mixture is in a range from 1 to 5 mol/L.

According to a preferred embodiment of this first aspect of the invention the random polymers are obtained by random polymerization of a cationic monomer and an anionic monomer and at least one of monomers other than the cationic and anionic monomers.

According to another embodiment of this first aspect of the invention the fabric is a woven fabric or unwoven fabric, it is in a form of a sheet, a tape, or a strip and the fabric is made of inorganic material, organic polymer material and a mixture thereof.

According to another embodiment of this first aspect of the invention the fabric is fully coated with the polyampholyte in a thickness of from 0.001mm to 10 mm.

In a second aspect the present invention discloses a method of preparation of a composite comprising a fabric and a polyampholyte hydrogel, comprising:
(a) providing a monomer mixture solution for preparation of a polyampholyte hydrogel,
   wherein the monomer mixture solution comprises at least one cationic monomer selected from the group consisting of 3-(methacryloylamino)propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC) and the anionic monomer sodium 4-styrenesulfonate (NaSS), and wherein the molar ratio of the anionic monomer to the total of the anionic monomer and a cationic monomer is in a range from 0.4 to 0.6, and the total concentration of the anionic and the cationic monomer in a monomer mixture is in a range from 1 to 5 mol/L;
(b) immersing a fabric in the monomer mixture solution; and
(c) polymerizing monomers in the monomer mixture solution to obtain a precursor of the composite.

According to an embodiment of this second aspect of the invention the monomer mixture solution further comprises a polymerization initiator and/or a cross-linking agent, or the monomer mixture solution further comprises a salt.

According to another embodiment of this second aspect of the invention the method further comprises a step (d) in which the precursor of the composite is desalted to obtain the composite.

According to another embodiment of this second aspect of the invention the fabric has a hydrophobic surface.

According to a third aspect of the present invention the composite of the first aspect of the invention is used in medical treatment, and preferably in ligament reconstruction surgeries and more preferably for Anterior Cruciate Ligament (ACL) reconstruction surgeries.

According to another aspect of the present invention the composite of the first aspect of the invention is used in bandages, skin grafts, flexible tear resistant clothing or bullet-proofing.

The present invention allows to make tear resistant materials that are extremely flexible, and are made of inexpensive materials. Although there is a large amount of prior work on blast resistant and tear resistant materials, there has not been any prior invention which claims to be extremely tear resistant, yet made of a hydrogel comprising water.

### Brief Description of The Drawings

The present invention will be described in the following text by the exemplary, non-limiting embodiments shown in the figures, wherein:
Figure 1 is a computer-aided drawing of one embodiment of the composites of the present invention. Left: a side view; right: a projection view.
Figure 2 is an image of testing setup for the tearing test.
Figure 3 is a tearing test result showing tear strength at breakage on one embodiment of the composite of the present invention. Comparison of a neat glass fiber fabric, a PA hydrogel, a combination of the fabric and a SN hydrogel, the SN hydrogel.
Figure 4 shows force vs. displacement curve for 20 mm wide samples of the composite of the present invention, a neat glass fiber fabric, a combination of the fabric and a SN hydrogel.
Figure 5 shows microscopy images of glass fiber fabric and PA hydrogel between the fibers of the fabric of one embodiment of the composite of the present invention.
Figure 6 is a microscopy image showing deformation in the tearing region of the fabric (macro scale)
Figure 7 is a tensile stress-strain measurement result on one embodiment of the composite of the present invention.

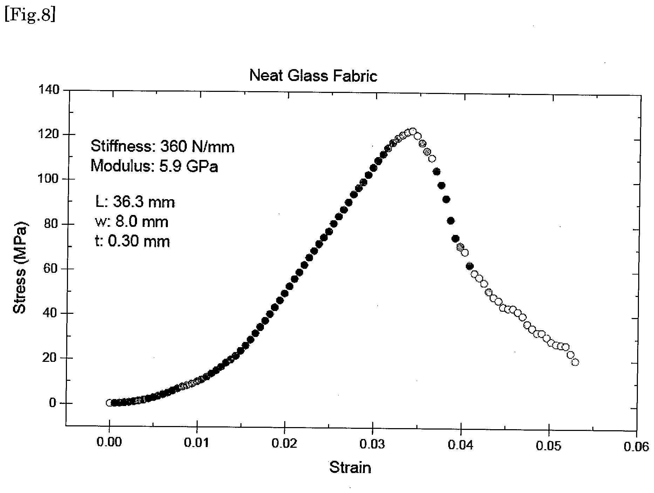

Figure 8 is a tensile stress-strain measurement result on a neat glass fabric.

### Descriptions of The Embodiments

The following preferred specific embodiments are, therefore, to be construed as merely illustrative, and not limitative of the remainder of the disclosure in any way whatsoever. In this regard, no attempt is made to show structural details of the present invention in more detail than is necessary for the fundamental understanding of the present invention, the description taken with the drawings making apparent to those skilled in the art how the several forms of the present invention may be embodied in practice.

This invention relates to a composite comprising a fabric and a PA hydrogel which is relatively soft and viscoelastic. The fabric can be a woven fabric or unwoven fabric and the unwoven fabric can be a knitted fabric. A woven fabric is obtained by producing the interlacing of warp fibers and weft fibers in weave styles, such as plain, twill, or satin styles and so on. A woven fabric is preferred because the integrity of the woven fabric is maintained by the mechanical interlocking of the fibers, and their drape, surface smoothness and stability can be controlled by the weave styles based on the design requirements.

The fabric can be made from inorganic material fibers, organic material fibers, biomaterial fibers or a mixture thereof. Inorganic material fibers are composed of inorganic chemical compounds, based on natural elements, which are resistant to high temperatures and high mechanical strength. Examples of the inorganic material fibers include glass fibers, carbon fibers, ceramics fibers and metals fibers. These inorganic materials fibers are commonly applied infields that require high performance materials, such as building materials, protective clothing, parts of airplanes, spaceships, and so on. Organic material fibers are made from synthesized polymers or small molecules, which are polymerized into long linear chains. Examples of the organic material fibers include synthetic polymer fibers such as nylon fibers, aramid fibers, polyalkylene fibers such as polyethylene fibers, polypropylene fibers. These organic material fibers with high mechanical performance and thermal stability are reinforced in the polymer matrix, which are applied in fields such as the automotive industry, railways, aerospace, and so on. Biomaterial fibers are made from plants and animals, which are low cost, low density, high toughness, renewable and biodegradable. Examples of biomaterial fibers include cellulose fibers and cotton fibers. These biomaterials fibers are widely reinforced with a polymer matrix in the textile, and automotive industries. The fabric is preferably comprised of long fibers. Woven and non-woven fabrics that are commercially available can be used. The fabric can be in a form of a sheet, a tape, a strip, a tube, a rod, and so on. The preferred composite is produced by the fabric with hydrophilic surface, such as glass fiber, and PA hydrogel.

In the composite of this invention, at least a part of the surface of the fabric is coated with the PA hydrogel. The PA hydrogel can be coated on either surface of the fabric or both surface of the fabric partially or fully. The PA hydrogel is a hydrogel of a polymer bearing randomly dispersed cationic and anionic repeat groups.

The PA hydrogel is selected from the group consisting of random polymers obtained by random polymerization of a cationic monomer and an anionic monomer. The cationic monomer is selected from the group consisting of 3-(methacryloylamino)propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC).

The anionic monomer is selected from sodium p-styrenesulfonate (NaSS).

One example of the PA hydrogels is P(NaSS-co-MPTC) hydrogels, randomly copolymerized from concentrated aqueous solutions of oppositely charged monomers, sodium p-styrenesulfonate (NaSS) and 3-(methacryloylamino)propyltrimethylammonium chloride (MPTC). One example of the P(NaSS-co-MPTC) hydrogels exhibits high strength (tensile fracture stress σb. 1.8 MPa), high extensibility (tensile fracture strain εb = 7.4), and high toughness (tearing energies T = 4000 J m⁻²). The PA hydrogels are self-recoverable due to the non-covalent ionic bonds. In addition to these excellent mechanical properties, these PA hydrogels have excellent biocompatibility and anti-biofouling properties. [NP-6].

Another example of a PA hydrogels is the PA P(NaSS-co-DMAEA-Q) hydrogels synthesized from sodium p-styrenesulfonate (NaSS) and acryloyloxethyltrimethylammonium chloride (DMAEA-Q). DMAEA-Q is relatively hydrophilic in comparison with the cationic monomer MPTC.

The chemical structures of monomers are shown below:
Cationic monomers: MPTC, DMAEA-Q;
Anionic monomers: NaSS.

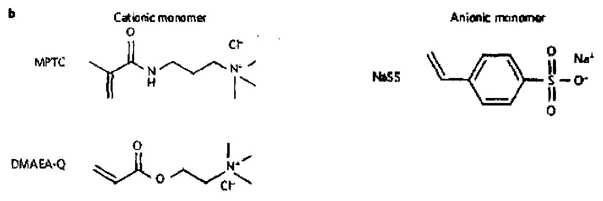

Other examples of PA hydrogels are the P(NaSS-co-MTAC4) and P(NaSS-co-VPC) systems synthesized by using methacrylatoethyl trimethyl ammonium chloride (MTAC) or 4-vinylpyridine chloride (4-VPC) as a cationic monomer. Chemical structures of the cationic monomers for the PA hydrogels are shown below:

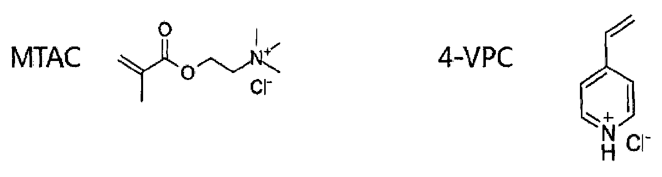

The above PAs are those developed from linear PAs by employing the ionic bond as a reversible sacrificial bond that breaks and reforms dynamically. [NP-6, 7] Owing to the random distribution, the opposite charges on the PAs form multiple ionic bonds of wide distribution in strength. The strong bonds work as permanent cross-linkers, imparting the elasticity of the hydrogel. The weak bonds are fragile and they break under stress, serving as reversible sacrificial bonds. Upon rupture of the weak bonds, the globule polymer chains unfolded, serving as hidden length. These two processes dissipate energy and impart toughness to the hydrogel. The PA hydrogels used in the present invention are supramolecular hydrogels and contain about 50 wt% water, yet are very tough and viscoelastic. An illustration of PA networks with ionic bonds of different strength is shown below. The strong bonds serve as permanent crosslinking points, and the weak bonds act as reversible sacrificial bonds that rupture under deformation.

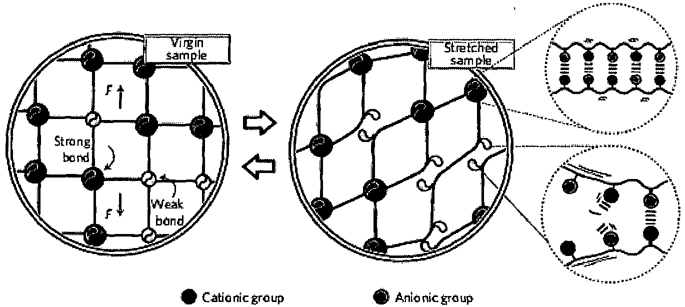

Neutral PAs form globule structures in aqueous medium by formation of ionic bonds, which leads to phase separation. It is better to determine the optimized charge ratio of the precursor solution to form the neutral PA. The effects of the monomer concentration Cm and the chemical cross-linker density C (CMBAA in case MBAA is used as the chemical cross-linker) on the behavior of the PA is considered when the PA hydrogel is prepared. These two parameters are crucial to control the phase separation of the system. It is preferred that a phase diagram of formulation in the Cm-CMBAA space can be constructed in advance and homogenous hydrogel phase is determined. The mechanical properties of PA hydrogels are dependent on the combinations of the ionic monomer pairs. There is a tendency of that more hydrophilic monomers produced softer and stretchable gels and vice versa.

The polymers are obtained by random copolymerization of a cationic monomer and an anionic monomer and a neutral monomer. The addition of the neutral monomer can adjust the stiffness and toughness of the samples. Examples of the neutral monomer include acrylamide based monomers such as acrylamide (AAm), N-isopropylacrylamide (NIPAM), dimethylacrylamide (DMAAm); vinyl based monomers such as vinyl acetate, vinyl pyridine, styrene; alkylacrylate based monomers such as methylmethacrylate; hydroxyalkyl acrylate based monomers such as hydroxyethylacrylate; and fluorine containing unsaturated monomers such as trifluoroethyl acrylate.

### Synthesis of PA hydrogels and preparation of the Composite

PA hydrogels are synthesized using a one-step random copolymerization of an anionic monomer and a cationic monomer. Using a one-step random copolymerization, the composite of the present invention can be manufactured in one step.

The present invention further discloses a method of preparation of a composite comprising a fabric and a polyampholyte hydrogel, comprising:
(a) providing a monomer mixture solution for preparation of a polyampholyte hydrogel,
   wherein the monomer mixture solution comprises at least one cationic monomer selected from the group consisting of 3-(methacryloylamino)propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC) and the anionic monomer sodium 4-styrenesulfonate (NaSS), and wherein the molar ratio of the anionic monomer to the total of the anionic monomer and a cationic monomer is in a range from 0.4 to 0.6, and the total concentration of the anionic and the cationic monomer in a monomer mixture is in a range from 1 to 5 mol/L;
(b) immersing a fabric in the monomer mixture solution; and
(c) polymerizing monomers in the monomer mixture solution to obtain a precursor of the composite.

### (a) Provision of a monomer mixture

The monomer mixture can be a mixed aqueous solution of an anionic monomer and a cationic monomer and the aqueous solution is prepared by mixing the monomers with water. The monomer concentration, Cm, influences the behavior of the resulting PA and the preparation of the PA hydrogels. The monomer concentration, Cm, means the total concentration of the anionic monomer and a cationic monomer. The monomer concentration, Cm, is according to the present invention in a range from 1 to 5 mol/L, preferably range from 1.5 to 2.5 mol/L in order to prepare PA hydrogels enabling to provide a tough and stiff, yet ' flexible composite.

In addition, molar fraction of the anionic monomer F influences the behavior of the resulting PA and the preparation of the PA hydrogels. The molar fraction of the anionic monomer F means a molar fraction of the anionic monomer to the total of the anionic monomer and a cationic monomer. The molar fraction of the anionic monomer F is according to the present invention in a range from 0.4 to 0.6, preferably in a range from 0.5 to 0.54 in order to prepare PA hydrogels that provide tough and stiff, yet flexible composites.

The mixed aqueous solution can further contain a polymerization initiator and/or a cross-linking agent and/or a salt. The polymerization initiator can be ultraviolet initiator such as 2-oxoglutaric acid, sodium 4-[2-(4-morpholino)benzoyl-2-dimethylamino]butylbenzenesulfonate, and phenacylpyridinium oxalate. The concentration of the polymerization initiator can be set in view of the kind of the monomers and the preferable initiator concentration is, but not limited to, in a range from 0.01mol% to 1mol% against to total monomer concentration.

The cross-linking agent is preferably a chemical cross-linker having two or more of (meth)acrylate groups in a molecule such as N, N'-methylenebis (acrylamide) (MBAA) and ethyleneglycoldimethacrylate. N, N'-methylenebis (acrylamide) (MBAA) is preferred when an (meth)acrylate based monomer is used. Ethyleneglycoldimethacrylate is preferred when a vinyl monomer such as NaSS and 4-VPC is used. The cross-linking agent can be used in combination or alone. The concentration of the cross-linking agent can vary in view of the monomers and the preferable concentration is, but not limited to, in a range of 0.1mol% to 10mol% against to total monomer concentration.

The salt can be an inorganic salt. Examples of the inorganic salt include an alkali metal salt such as LiCl, NaCl, KCl, CsCl, CaCl₂, MgSO₄, K₂SO₄, or MgCl₂. The concentration of the salt can vary in view of the kind of the monomers and the monomer concentration Cm and is not limited specifically but is in a range of 0.1M to 2M, for example. The usage of salt can reduce the viscosity of the mixed aqueous solution and obtain the homogeneous samples. However, the higher the salt concentration, the higher the burden of removal of the salt after polymerization.

### (b) Immersion of a fabric into the monomer mixture solution;

A cell for preparation of the composite is comprised of, but not limited to, a pair of plates such as glass plates facing each other and the thickness between the plates is controlled by a spacer thickness. This spacing is, but not limited to, desirably in a range from 0.001 mm to 10 mm, preferably in a range from 0.01 mm to 5.0 mm, and more preferably in a range from 0.1 mm to 1.0 mm. The plates and the spacer are selected from the material inert with the mixed aqueous solution and the spacer can be a silicone spacer, for example, with a prescribed thickness. The thickness of the spacer decides the thickness of the composite. A fabric is placed in the space made by the plates. This fabric can be woven or knit or unwoven and may range in thickness from 0.001 mm to 1 mm in thickness, and may consist of a plain, twill, or satin weave. The mixed aqueous solution is added into the channel in which the fabric has been placed. The fabric can be placed nominally in the center of the space, or preferentially towards an edge or surface such as 60/40, 70/30, 80/20, etc. distribution of the PA on each side of the fabric. Alternatively, the composite may be prepared by coating the fabric with the mixed aqueous solution on either or both sides of the fabric surface. The coating may be applied by spraying, dipping, knife-over-roll, or other traditional fabric coating methods.

### (c) Polymerization of the monomers to obtain a precursor of the composite

The mixed aqueous solution is cured, for example, by irradiation of ultraviolet light when an ultraviolet initiator is used. Wavelength of the ultraviolet light can be determined in view of the ultraviolet initiator. The irradiation can be conducted in an inert atmosphere, such as argon atmosphere, at an ambient temperature or an increased temperature for 1 minute to 24 hours, preferably from 1 hour to 12 hours, and more preferably from 8 hours to 12 hours, which is adjusted by the power of ultraviolet light. This reaction can take place at an ambient temperature or an increased temperature in an inert atmosphere such as an argon or nitrogen atmosphere. The composite consisted of fabric and PA can be prepared with the thickness from 0.001mm to 10mm controlled by the size of spacer.

In this method, the cationic and anionic monomers are essentially copolymerized randomly in the PA hydrogels. The random structure of the copolymer can be confirmed using a ¹H-NMR reaction kinetics study, for example.

After polymerization, the as-prepared gel is immersed in water to reach equilibrium and to wash away the residual chemicals after one week, for example. The amount of the water is not limited specifically and it is preferred to confirm the equilibrium state and disappearance of the residual chemicals in the gel. During this process, the mobile counter-ions of the ionic copolymer as well as the salt used, are removed from the gel, and the oppositely charged ions on the copolymer form stable ionic complexes either through intra- or interchain interactions.

Polymerization conditions and structural features and mechanical properties of various PA hydrogels are shown in the table below.

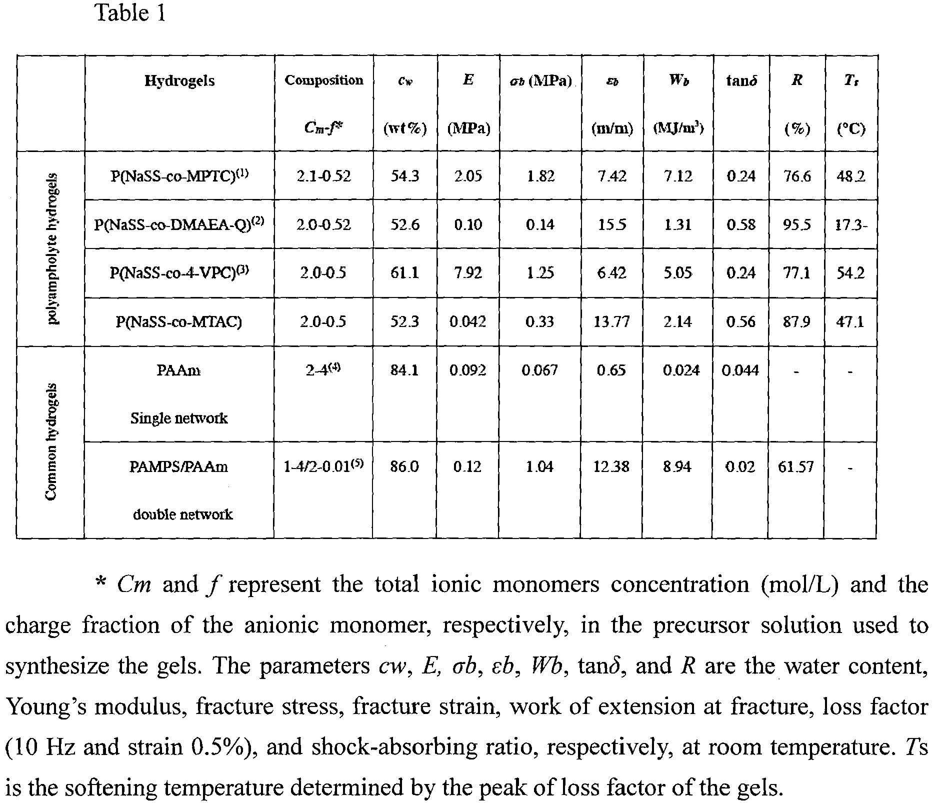

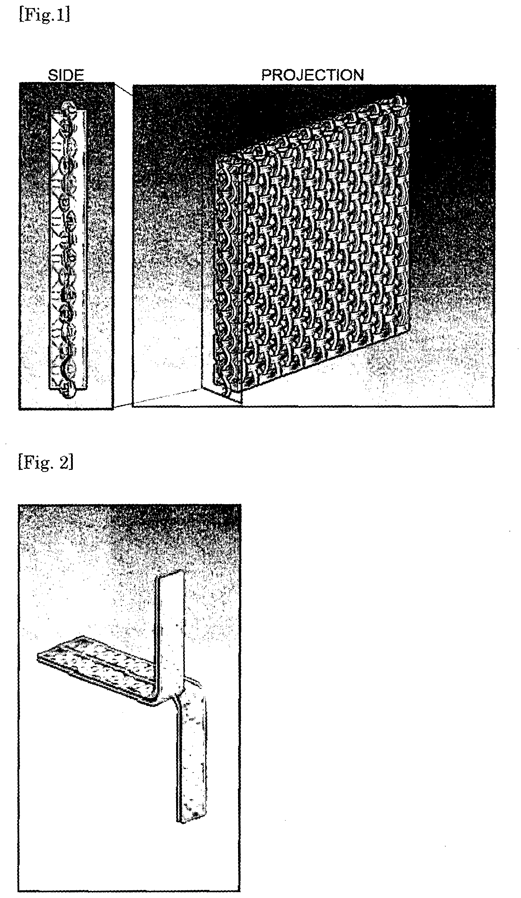

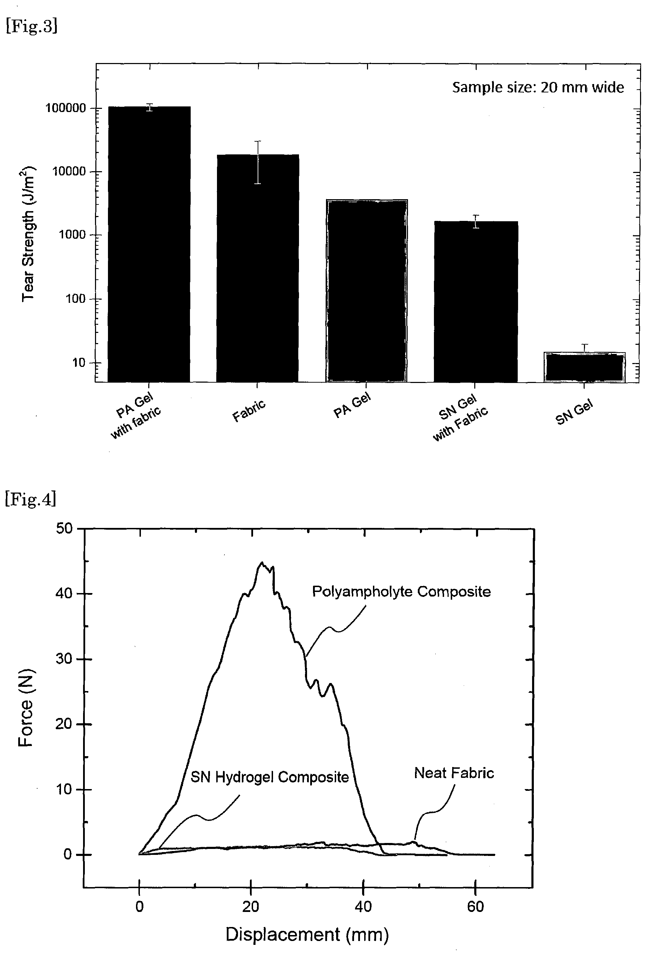

A computer-aided drawing of one embodiment of the composites of the present invention is shown in Figure 1. Left side is a side view of the composite and right side is a projection view. One embodiment of the composites of the present invention exhibits toughness two orders of magnitude higher than traditional hydrogels (SN gel) (Gc 4800 J/m² vs 10 J/m²). In addition, one embodiment of the composites of present invention exhibits dramatic increase in toughness. For the case of a 20 mm wide strip of the composite comprised of woven glass fiber and PA gel, a Gc value (105,000 J/m²) can be obtained which is an additional two orders of magnitude greater than the neat PA gel, and 4 orders of magnitude higher than a traditional SN gel. (See Figure 3).

Interestingly, unlike most composites where combining two materials results in an averaging of their mechanical properties, in this case the tear strength is much greater than either materials when tested independently. Also when the control SN gel and fabric was tested, a noticeable decrease in tear strength was observed, which reinforces that this special combination of PA gel and fabric is necessary to see a great increase in tear strength (See the following Examples).

The composite of the present invention can be employed for medical treatment for example for ligament reconstruction surgeries such as Anterior Cruciate Ligament (ACL) reconstruction surgeries. The composite of the present invention can also be employed for bandages, skin grafts, flexible tear resistant clothing or bullet-proofing.

There are many ways this invention improves over the prior art. When used as internal prostheses, fabrics have many issues with regards to tearing, failing, and degrading. Often to prevent this, these stiff substrates would be coated in elastomers. However elastomers often result in a bodily response which can lead to infection or rejection. The coating material, the PA hydrogel, used here contains primarily of water and is non-toxic, and resists cell adhesion.

The composite of the present invention has various utilities. Wound care would benefit from this invention, because the PA hydrogels could create extremely resilient and tear resistant bandages. In the composite of the present invention, the fabric is used as support and the PA hydrogel is used for wound closure, as they are generally non-toxic, consist mostly of water, and can promote wound healing.

### Examples

The present invention will be described in detail below based on examples. However, the present invention is not limited to the examples.

### 1. Materials

Sodium p-styrenesulfonate (NaSS), Methyl chloride quarternized N, N-dimethylamino ethylacrylate (DMAEA-Q), Acrylamide (AAm), 2-oxoglutaric acid and N, N'-methylenebis (acrylamide) (MBAA) were purchased from the Wako company (Japan). Fabric (S-Glass 4-Harness Satin Weave) (4HS) were purchased from the ACP composite company. In the 4HS, the fill yarn passes over three warp yarns and under one. All of the chemicals were used as purchased without further purification.

### 2. Methods

### 2-1. Synthesis of materials

Polyampholyte hydrogels (PA) were synthesized using the one-step random copolymerization of an anionic monomer NaSS and a cationic monomer DMAEA-Q. A mixed aqueous solution with the prescribed monomer concentration (Cm=2.0 mol/L) and molar fraction of the anionic monomer (F=0.52), 0.1mol% ultraviolet initiator, 2-oxoglutaric acid, 0.1mol% chemical crosslinker MBAA (in a concentration relative to the total monomer concentration), and 0.5M NaCl was poured into a reaction cell. The cell was consisted of a pair of glass plates with 10 mm (L)×10 mm (w)×1mm(d), where the thickness was controlled by the silicone spacer thickness. The reacted system was irradiated with 365-nm ultraviolet light for 8h in the atmosphere of argon.

Polyacrylamide hydrogels (PAAm) were produced from the polymerization of 2 mol/L acrylamide monomer, 0.1mol% 2-oxoglutaric acid and 1.0mol% chemical crosslinker MBAA (relative to the total monomer concentration) under the UV light.

PA gel/fabric composite were produced by the similar described polymerization process. The fabric 4HS with approximately 0.3 mm thickness were fixed to the center of reaction cell prior to injecting the reaction solution. To produce the PA gel/fabric composite, the polymerization was carried out after filling the PA solution with the same composition to the reaction cell.

Similar to the PA gel/fabric composite production, the PAAm/fabric composite was synthesized by the AAm solution and fabric 4HS.

After polymerization, the as-prepared gel was immersed in a large amount of water for 1 week to allow the gel to reach equilibrium and to wash away the residual chemicals and counter-ions.

### 2-2. Characterization

### Tearing test:

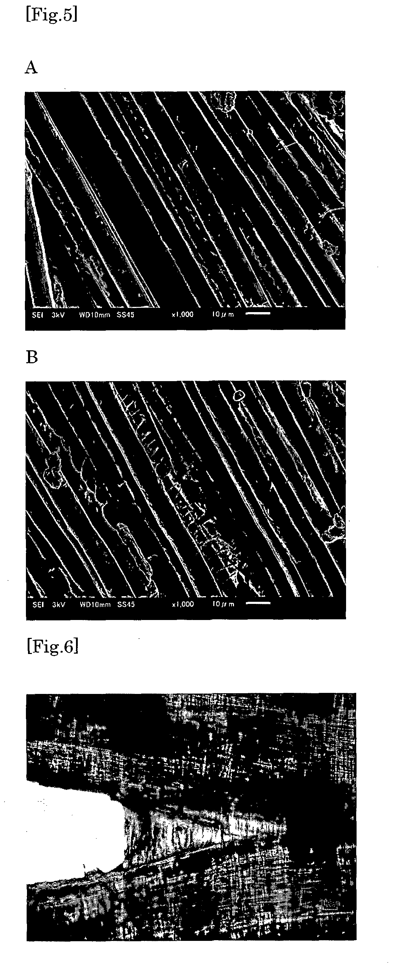

The tearing test was performed to characterize the toughness in air using a commercial test machine (Instron Cat. No 2752-005). The image of testing setup for the tearing test is shown in Figure 2. The test samples were cut to rectangular shape with 40 mm (L) × 20-100 mm (w) × 0.5-1.3 mm (t). A cut was made 20 mm along the length, at the center of the width of the sample, to create two arms. The two arms of a test piece were clamped, and then the upper arm was pulled upward at constant velocity 50 mm/min while the tearing force F was recorded. The tearing energy T was calculated at a constant tearing force F using the relation T = 2F/w, where w is the thickness of the sample. The results are shown in Figure 3 and 4. Figure 3 shows tear strength at breakage and Figure 4 shows force vs. displacement curve for 20 mm wide samples of the composite of the present invention, a neat glass fiber fabric, a combination of the fabric and a SN hydrogel. Figure 5 shows microscopy images of glass fiber fabric and PA hydrogel between the fibers of the fabric of the sample, A is an image of fibrillation in the matrix gel and B is an image of fracture of the fibrils, which is hypothesized to dissipate energy. Figure 6 is a microscopy image showing deformation in the tearing region of the fabric (macro scale).

### Tensile test:

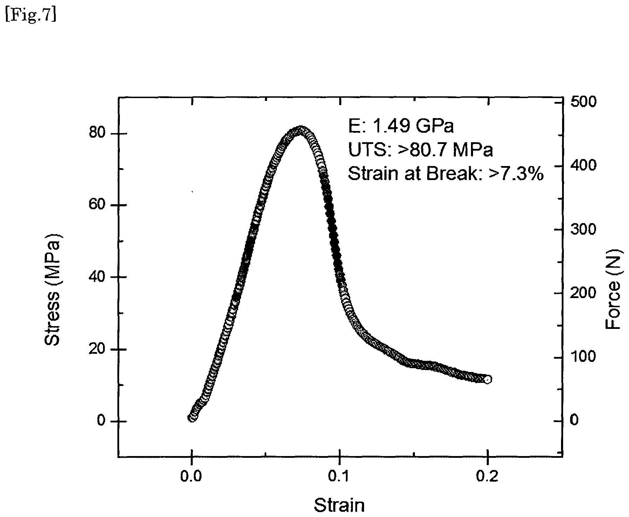

The tensile stress-strain measurements were performed using a tensile-compressive tester (Tensilon RTC-1310A, Orientec Co.) at a deformation rate of 10 mm/min in air. The test was carried out on samples with rectangle shape with the size (25 mm (L) × 20 mm (w) × 0.85 mm (d). In order to prevent the slipping of the sample at the end of the clamp during the tensile test, polyethylene terephthalate (PET) plates with 2 mm (L) × 2 mm (w) × 3mm (d) adhere the ends of samples was used by super glue cyanoacrylate. The PET plates were gripped by the clamp during the tensile test. The results are shown in Figures 7 (composite) and 8 (neat glass fabric). The strength of the materials exceeded the strength of the mounting grips, and the ultimate properties of these materials exceed the results presented.

Although the present invention has been described in considerable detail with regard to certain versions thereof, other versions are possible, and alterations, permutations and equivalents of the version shown will become apparent to those skilled in the art upon a reading of the specification and study of the drawings. Also, the various features of the versions herein can be combined in various ways to provide additional versions of the present invention. Furthermore, certain terminology has been used for the purposes of descriptive clarity, and not to limit the present invention. Therefore, any appended claims should not be limited to the description of the preferred versions contained herein and should include all such alterations, permutations, and equivalents as fall within the true spirit and scope of the present invention.

Having now fully described this invention, it will be understood to those of ordinary skill in the art that the methods of the present invention can be carried out with a wide and equivalent range of conditions, formulations, and other parameters without departing from the scope of the invention or any embodiments thereof.

The citation of any publication is for its disclosure prior to the filing date and should not be construed as an admission that such publication is prior art or that the present invention is not entitled to antedate such publication by virtue of prior invention.

## Claims

1. A composite comprising a fabric and a polyampholyte hydrogel, wherein the polyampholyte hydrogel is a hydrogel of a polymer bearing randomly dispersed cationic and anionic repeat groups; and at least a part of the fabric is coated with the polyampholyte hydrogel, wherein the polyampholyte hydrogel is selected from the group consisting of random polymers obtained by random polymerization of at least one cationic monomer selected from the group consisting of 3-(methacryloylamino)-propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC), and the anionic monomer sodium p-styrenesulfonate (NaSS), and wherein the molar fraction of the anionic monomer to the total of the anionic monomer and the cationic monomer is in a range from 0.4 to 0.6 and the total concentration of the anionic monomer and the cationic monomer in a monomer mixture is in a range from 1 to 5 mol/L.

2. The composite according to claim 1, wherein the random polymers are obtained by random polymerization of a cationic monomer and an anionic monomer and at least one of monomers other than the cationic and anionic monomers.

3. The composite according to claim 1, wherein the fabric is a woven fabric or unwoven fabric, it is in a form of a sheet, a tape or a strip, and the fabric is made from inorganic material fibers, organic material fibers, biomaterial fibers or a mixture thereof.

4. The composite according to claim 1, wherein the fabric is fully coated with the polyampholyte in a thickness of from 0.001 mm to 10 mm.

5. A method of preparation of a composite comprising a fabric and a polyampholyte hydrogel, comprising:
(a) providing a monomer mixture solution for preparation of a polyampholyte hydrogel, wherein the monomer mixture solution comprises at least one cationic monomer selected from the group consisting of 3-(methacryloylamino)-propyltrimethylammonium chloride (MPTC), acryloyloxethyltrimethylammonium chloride (DMAEA-Q), methacrylatoethyl trimethyl ammonium chloride (MTAC) and 4-vinylpyridine chloride (4-VPC), and the anionic monomer sodium p-styrenesulfonate (NaSS), and wherein a molar fraction of the anionic monomer to the total of the anionic monomer and the cationic monomer is in a range from 0.4 to 0.6 and the total concentration of the anionic monomer and the cationic monomer in a monomer mixture is in a range from 1 to 5 mol/L;
(b) immersing a fabric in the monomer mixture solution; and
(c) polymerizing monomers in the monomer mixture solution to obtain a precursor of the composite.

6. The method according to claim 5, wherein the monomer mixture solution further comprises a polymerization initiator and/or a cross-linking agent, or the monomer mixture solution further comprises a salt.

7. The method according to claim 5, wherein the method further comprises a step (d) in which the precursor of the composite is desalted to obtain the composite.

8. The method according to claim 5, wherein the fabric has a hydrophobic surface.

9. Use of the composite of claim 1 in medical treatment.

10. Use of the composite of claim 1 in ligament reconstruction surgeries, preferably Anterior Cruciate Ligament (ACL) reconstruction surgeries.

11. Use of the composite of claim 1 in bandages, skin grafts, flexible tear resistant clothing or bullet-proofing.

## Patentansprüche

1. Verbundstoff mit Gewebe und Polyampholyt-Hydrogel, wobei das Polyampholyt-Hydrogel ein Hydrogel aus einem Polymer ist, das statistisch verteilte kationische und anionische Wiederholungsgruppen aufweist; und wenigstens ein Teil des Gewebes mit dem Polyampholyt-Hydrogel beschichtet ist, wobei das Polyampholyt-Hydrogel ausgewählt ist aus der Gruppe bestehend aus statistischen Polymeren, die durch statistische Polymerisation wenigstens eines kationischen Monomers erhalten sind, ausgewählt aus der Gruppe bestehend aus 3-(Methacryloylamino)-Propyltrimethylammoniumchlorid (MPTC), Acryloyloxethyltrimethylammoniumchlorid (DMAEA-Q), Methacrylatoethyl-Trimethylammoniumchlorid (MTAC) und 4-Vinylpyridinchlorid (4-VPC) und dem anionischen Monomer Natrium-p-styrolsulfonat (NaSS), und wobei der Molenbruch des anionischen Monomers zur Gesamtheit der anionischen Monomere und kationischen Monomere im Bereich von 0,4 bis 0,6 und die Gesamtkonzentration der anionischen Monomere und kationischen Monomere in einem Monomergemisch im Bereich von 1 bis 5 mol/L liegt.

2. Zusammensetzung nach Anspruch 1, wobei die statistischen Polymere aus der statistischen Polymerisation eines kationischen Monomers und eines anionischen Monomers erhalten sind und wenigstens eines der Monomere von den kationischen und anionischen Monomeren verschieden ist.

3. Zusammensetzung nach Anspruch 1, wobei das Gewebe ein gewebtes oder ungewebtes Gewebe ist, in Form einer Bahn, eines Bandes oder eines Streifens vorliegt und das Gewebe aus anorganischen Materialfasern, organischen Materialfasern, Biomaterialfasern oder einem Gemische davon hergestellt ist.

4. Zusammensetzung nach Anspruch 1, wobei das Gewebe vollständig mit dem Polyampholyt in einer Dicke von 0,001 min bis 10 min beschichtet ist.

5. Verfahren zur Herstellung einer Zusammensetzung aus einem Gewebe und einem Polyampholyt-Hydrogel, umfassend:
(a) Bereitstellen einer Monomergemischlösung zur Herstellung eines Polyampholyt-Hydrogels, Wobei die Monomergemischlösung wenigstens ein kationisches Monomer umfasst, ausgewählt aus der Gruppe bestehend aus 3-(Methacryloylamino)-Propyltrimethylammoniumchlorid (MPTC), Acryloyloxethyltrimethylammoniumchlorid (DMAEA-Q), Methacrylatoethyl-Trimethylammoniumchlorid (MTAC) und 4-Vinylpyridinchlorid (4-VPC) und dem anionischen Monomer Natrium-p-styrolsulfonat (NaSS), und wobei ein Molenbruch des anionischen Monomers zur Gesamtheit der anionischen Monomere und kationischen Monomere im Bereich von 0,4 bis 0,6 und die Gesamtkonzentration der anionischen Monomere und kationischen Monomere in einem Monomergemisch R im Bereich von 1 bis 5 mol/L liegt;
(b) Eintauchen des Gewebes in die Monomergemischlösung; und
(c) Polymerisieren der Monomere in der Monomergemischlösung, um eine Vorstufe der Zusammensetzung zu erhalten.

6. Verfahren nach Anspruch 5, wobei die Monomergemischlösung ferner einen Polymerisationsinitiator und/oder ein Vernetzungsmittel umfasst oder die Monomergemischlösung ferner ein Salz umfasst.

7. Verfahren nach Anspruch 5, wobei das Verfahren weiter einen Schritt (d) umfasst, in dem die Vorstufe der Zusammensetzung entsalzt wird, um die Zusammensetzung zu erhalten.

8. Verfahren nach Anspruch 5, wobei das Gewebe eine hydrophobe Oberfläche aufweist.

9. Verwendung der Zusammensetzung nach Anspruch 1 zur medizinischen Behandlung.

10. Verwendung der Zusammensetzung nach Anspruch 1 bei Bandrekonstruktionseingriffen, vorzugsweise Eingriffen zur Rekonstruktion des vorderen Kreuzbandes.

11. Verwendung der Zusammensetzung nach Anspruch 1 bei Bandagen, Hauttransplantationen, in flexibler, reißfester Kleidung oder Schusshemmung.

## Revendications

1. Composite comprenant un tissu et un hydrogel de polyampholyte, dans lequel l'hydrogel de polyampholyte est un hydrogel d'un polymère portant des groupes de répétition cationiques et anioniques dispersés de manière aléatoire ; et au moins une partie du tissu est revêtue de l'hydrogel de polyampholyte, dans lequel l'hydrogel de polyampholyte est choisi dans le groupe des polymères statistiques obtenus par polymérisation statistique d'au moins un monomère cationique choisi dans le groupe constitué du chlorure de 3-(méthacryloylamino)-propyltriméthylammonium (MPTC), du chlorure d'acryloyloxéthyltriméthylammonium (DMAEA-Q), du chlorure de méthacrylatoéthyl triméthyl ammonium (MTAC) et du chlorure de 4-vinylpyridine (4-VPC) et le monomère anionique p-styrènesulfonate de sodium (NaSS), et dans lequel la fraction molaire du monomère anionique par rapport au total du monomère anionique et du monomère cationique est comprise entre 0,4 et 0,6 et la concentration totale du monomère anionique et du monomère cationique dans un mélange de monomères est comprise entre 1 et 5 mol/L.

2. Composite selon la revendication 1, dans lequel les polymères statistiques sont obtenus par polymérisation statistique d'un monomère cationique et d'un monomère anionique et d'au moins l'un des monomères autres que les monomères cationiques et anioniques.

3. Composite selon la revendication 1, dans lequel le tissu est un tissu tissé ou non tissé, il se présente sous la forme d'une feuille, d'un ruban ou d'une bande, e le tissu est constitué de fibres de matériau inorganique, de fibres de matériau organique, de fibres de biomatériau ou d'un mélange de ceux-ci.

4. Composite selon la revendication 1, dans lequel le tissu est entièrement recouvert du polyampholyte en une épaisseur de 0,001 min à 10 min.

5. Procédé de préparation d'un composite comprenant un tissu et un hydrogel de polyampholyte, comprenant les étapes consistant à :
(a) fournir une solution de mélange de monomères pour la préparation d'un hydrogel de polyampholyte, dans laquelle la solution de mélange de monomères comprend au moins un monomère cationique choisi dans le groupe constitué du chlorure de 3-(méthacryloylamino)-propyltriméthylammonium (MPTC), du chlorure d'acryloyloxéthyltriméthylammonium (DMAEA-Q), du chlorure de méthacrylatoéthyl triméthyl ammonium (MTAC) et du chlorure de 4-vinylpyridine (4-VPC), et le monomère anionique p-styrènesulfonate de sodium (NaSS), et dans laquelle une fraction molaire du monomère anionique par rapport au total du monomère anionique et du monomère cationique est comprise entre 0,4 et 0,6 et la concentration totale du monomère anionique et du monomère cationique dans un mélange de monomères R varie entre 1 et 5 mol/L ;
(b) immerger un tissu dans la solution de mélange de monomères ; et
(c) polymériser des monomères dans la solution de mélange de monomères pour obtenir un précurseur du composite.

6. Procédé selon la revendication 5, dans lequel la solution de mélange de monomères comprend en outre un initiateur de polymérisation et/ou un agent réticulant, ou la solution de mélange de monomères comprend en outre un sel.

7. Procédé selon la revendication 5, dans lequel le procédé comprend en outre une étape (d) dans laquelle le précurseur du composite est dessalé pour obtenir le composite.

8. Procédé selon la revendication 5, dans lequel le tissu a une surface hydrophobe.

9. Utilisation du composite de la revendication 1 dans le traitement médical.

10. Utilisation du composite de la revendication 1 dans des chirurgies de reconstruction de ligament, de préférence des chirurgies de reconstruction du ligament croisé antérieur (LCA).

11. Utilisation du composite de la revendication 1 dans des pansements, des greffes de peau, des vêtements résistants à la déchirure ou des pare-balles.
